(19) Europäisches Patentamt
European Patent Office
Office européen des brevets

(11) Numéro de publication : **0 022 387**
**B1**

(12) # FASCICULE DE BREVET EUROPÉEN

(45) Date de publication du fascicule du brevet : **05.01.83**

(21) Numéro de dépôt : **80400856.3**

(22) Date de dépôt : **13.06.80**

(51) Int. Cl.[3] : **C 07 C 41/16**, C 07 C 79/355, C 07 C 87/60, C 07 C149/32, C 07 C121/75, C 07 C 87/123

(54) **Procédé de préparation de dérivés benzéniques par réaction d'un halogénobenzène activé avec un réactif organique anionique oxygéné ou soufré.**

(30) Priorité : **27.06.79 FR 7916544**

(43) Date de publication de la demande : **14.01.81 Bulletin 81/02**

(45) Mention de la délivrance du brevet : **05.01.83 Bulletin 83/01**

(84) Etats contractants désignés : **AT BE CH DE FR GB IT LI LU NL SE**

(56) Documents cités : **NEANT**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Soula, Gérard**
**33, Rue Nungesser**
**F-69330 Meyzieu (FR)**

(74) Mandataire : **Cazes, Jean-Marie et al**
**RHONE-POULENC RECHERCHES Service Brevets Chimie et Polymères 25, quai Paul Doumer**
**F-92408 Courbevoie Cedex (FR)**

**EP 0 022 387 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Procédé de préparation de dérivés benzéniques par réaction d'un halogénobenzène activé avec un réactif organique anionique oxygéné ou soufré

La présente invention concerne un procédé de préparation de dérivés benzéniques ; elle concerne plus particulièrement un procédé de préparation de dérivés benzéniques par réaction d'un halogénobenzène activé avec un réactif organique anionique oxygéné ou soufré. L'invention concerne également les produits obtenus par la mise en œuvre de ce procédé.

On entend par halogénobenzène activé un halogénobenzène comportant en ortho ou para de l'halogène un groupement électroattracteur et par réactif organique anionique oxygéné ou soufré un réactif du type $RO^-M^+$ ou $RS^-M^+$, R étant un reste hydrocarboné.

On connaît dans l'art antérieur la demande de brevet français 76.13943 publiée sous le numéro 2.311.004. Ce document décrit un procédé de préparation de composés de formule générale A—Y—A'—Zn dans laquelle A et A' représentent des restes aryliques substitués ou non substitués, Z est un groupement fixant les électrons et Y est O, S ou $SO_2$, n étant compris entre 1 et 3. Selon ce procédé, on fait réagir un composé de formule A—YMe où Me représente un métal alcalin ou $NH_4$ avec un composé de formule X—A'—Zn dans laquelle X est un halogène ou un groupe nitroactivé.

La réaction est réalisée dans un système à deux phases : l'une étant de l'eau ou un milieu aqueux alcalin dans laquelle le composé A—YMe est mis en réaction, l'autre étant constituée par le composé X—A'—Zn en solution dans un ou des solvants non miscibles à l'eau. La réaction est effectuée en présence de dérivés d'ammonium ou de phosphonium quaternaires comme catalyseurs.

Les principaux inconvénients de ce type de procédé sont liés à l'utilisation d'une phase aqueuse. La présence d'eau implique de travailler sous pression lorsque la température de réaction est supérieure à 100 °C. De plus, elle entraîne la mise en œuvre de solvants non miscibles à l'eau et ne formant pas d'émulsions avec l'eau en présence de dérivés d'ammonium quaternaires. Or, certaines réactions ne s'effectuent avec un rendement appréciable que dans des solvants polaires aprotiques tels que le sulfolane, le DMSO et la N-méthylpyrolidone qui sont des solvants miscibles à l'eau. Il faut également mentionner que la grande quantité d'eau utilisée, telle qu'elle ressort des exemples de la demande de brevet français précitée, implique l'utilisation de réacteurs importants.

D'autres inconvénients résultent de l'utilisation comme catalyseurs de dérivés d'ammonium ou de phosphonium quaternaires. En effet, l'homme de l'art sait bien que ce sont des produits qui se dégradent facilement lorsque la température est supérieure à environ 130 °C. De plus, de sérieuses difficultés sont rencontrées au plan industriel pour séparer le catalyseur du produit de la réaction.

Un inconvénient supplémentaire de ce type de procédé de l'art antérieur réside dans le fait qu'il ne permet pas la mise en œuvre d'alcoolates qui se dégradent en présence d'eau.

La demanderesse a découvert un procédé qui pallie les inconvénients de l'art antérieur.

L'invention a pour objet un procédé de préparation de composés benzéniques par réaction d'un halogénobenzène activé avec un réactif organique anionique oxygéné ou soufré mis en œuvre en l'absence d'eau.

Un autre objet de l'invention est un tel procédé mettant en œuvre un catalyseur ne se dégradant pas à température élevée.

Un autre objet de l'invention est un tel procédé permettant une séparation et une récupération aisée du catalyseur.

La présente invention concerne donc un procédé de préparation de composés benzéniques par réaction d'un halogénobenzène activé avec un réactif organique anionique oxygéné ou soufré caractérisé en ce que la réaction a lieu en présence d'au moins un agent séquestrant de formule :

$$N—[CHR_1—CHR_2—O—(CHR_3—CHR_4—O)_n—R_5]_3 \qquad (I)$$

dans laquelle n est un nombre entier supérieur ou égal à 0 est inférieur ou égal à 10 ($0 \leq n \leq 10$), $R_1$,$R_2$, $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $—C_mH_{2m}—C_6H_5$ ou $C_mH_{2m+1}—C_6H_4—$, où m est compris entre 1 et 12.

On peut opérer en présence ou en l'absence de solvant. Lorsqu'on n'utilise pas de tiers solvant, c'est l'halogénobenzène activé lui-même qui joue le rôle de solvant.

L'invention repose sur le fait que l'agent séquestrant de formule (I) forme avec le réactif organique anionique oxygéné ou soufré un complexe qui est soluble dans des solvants dans lesquels le réactif organique anionique oxygéné ou soufré est insoluble ou très peu soluble à l'état non complexé. Il en résulte clairement que le procédé selon l'invention donne la possibilité de mettre en œuvre des solvants dont l'utilisation n'était pas envisageable auparavant. Ceci est d'autant plus avantageux qu'il devient possible d'utiliser des solvants dont la manipulation à l'échelle industrielle est beaucoup plus aisée que celle de ceux utilisés auparavant. Un autre avantage de l'invention est que, bien que cela ne soit pas complètement expliqué, il apparaît que la complexation due à l'agent séquestrant de formule (I) active le système réactionnel.

Selon un mode de réalisation préférentiel de l'invention, on utilise un agent séquestrant de formule (I)

dans laquelle $R_1$, $R_2$, $R_3$ et $R_4$ représentent un atome d'hydrogène ou un radical méthyle, $R_5$ et n ayant la signification précédente.

Parmi ces derniers, on préfère encore plus particulièrement mettre en œuvre les agents séquestrants pour lesquels n est supérieur ou égal à 0 et inférieur ou égal à 6 pour lesquels $R_5$ représente un radical alkyle ayant de 1 à 4 atomes de carbone.

On peut citer :

— la tris(oxa-3 butyl)amine de formule :
$N—(CH_2—CH_2—O—CH_3)_3$
— la tris(dioxa-3, 6 heptyl)amine de formule :
$N—(CH_2—CH_2—O—CH_2—CH_2—O—CH_3)_3$
— la tris(trioxa-3, 6, 9 décyl)amine de formule :
$N—(CH_2—CH_2—O—CH_2—CH_2—O—CH_2—CH_2—O—CH_3)_3$
— la tris(dioxa-3, 6 octyl)amine de formule :
$N—(CH_2—CH_2—O—CH_2—CH_2—O—C_2H_5)_3$
— la tris(trioxa-3, 6, 9 undécyl)amine de formule :
$N—(CH_2—CH_2—O—CH_2—CH_2—O—CH_2—CH_2—O—C_2H_5)_3$
— la tris(dioxa-3, 6 nonyl)amine de formule :
$N—(CH_2—CH_2—O—CH_2—CH_2—O—C_3H_7)_3$
— la tris(trioxa-3, 6, 9 dodécyl)amine de formule :
$N—(CH_2—CH_2—O—CH_2—CH_2—O—CH_2—CH_2—O—C_3H_7)_3$
— la tris(dioxa-3, 6 décyl)amine de formule :
$N—(CH_2—CH_2—O—CH_2—CH_2—O—C_4H_9)_3$
— la tris(trioxa-3, 6, 9 tridécyl)amine de formule :
$N—(CH_2—CH_2—O—CH_2—CH_2—C—CH_2—CH_2—O—C_4H_9)_3$
— la tris(tétraoxa-3, 6, 9, 12 tridecyl)amine de formule :
$N—[CH_2—CH_2—O\text{-}(—CH_2—CH_2—O—)\text{-}_3CH_3]_3$
— la tris(hexaoxa-3, 6, 9, 12, 15, 18 nonadécyl)amine de formule :
$N—[CH_2—CH_2—O\text{-}(—CH_2—CH_2—O—)\text{-}_5CH_3]_3$
— la tris(dioxa-3, 6 méthyl-4 heptyl)amine de formule :
$N—[CH_2—CH_2—O—CH(CH_3)—CH_2—O—CH_3]_3$
— la tris(dioxa-3, 6 diméthyl-2, 4 heptyl)amine de formule :
$N—[CH_2—CH(CH_3)—O—CH(CH_3)CH_2—O—CH_3]_3$

Les amines utilisées dans le procédé selon l'invention sont connues en tant que telles dans l'art antérieur. C'est ainsi que le brevet français 1 302 365 cite l'obtention des amines tertiaires $N(CH_2—CH_2—O—CH_3)_3$ et $N—(CH_2—CH_2—O—CH_2—CH_2—O—CH_3)_3$ comme sous-produits de la synthèse des amines primaires et secondaires correspondantes, ces amines primaires et secondaires étant des produits intéressants comme intermédiaires en vue de la synthèse de substances pharmaceutiques, comme inhibiteurs de corrosion, comme intermédiaires en vue de la synthèse de produits chimiques intéressants en agriculture et comme émulsifiants. Il n'est pas inutile de souligner que le domaine d'application des composés obtenus dans le brevet 1 302 365 précité simultanément aux amines utilisées dans le procédé, objet de la présente demande, est totalement étranger au domaine de l'invention.

Le procédé selon l'invention est applicable à la réaction d'un halogénobenzène activé de formule générale :

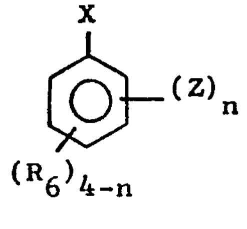 (II)

dans laquelle :

X représente un atome d'halogène (F, Cl, Br ou I),

Z représente au moins un groupement électroattracteur choisi parmi le groupe comprenant $NO_2$, CN, $SO_3M$, $CO_2M$, $CF_3$ où M représente un métal alcalin, Z étant situé en ortho et/ou para du groupement X,

$R_6$ représente au moins un élément choisi parmi le groupe comprenant :

l'hydrogène,

les radicaux alkyle et cycloalkyle ayant de 1 à 12 atomes de carbone,

les radicaux alcényle ayant de 3 à 12 atomes de carbone comme les radicaux propényle, nonyle, dodécyle par exemple

les radicaux de formule $C_mH_{2m+1}—C_6H_4—$ ; $C_mH_{2m-1}—C_6H_4$ ; et $C_6H_5—C_mH_{2m}—$ où m est un nombre entier compris entre 1 et 12 ($1 \leq m \leq 12$) et où $C_6H_4$ et $C_6H_5$ peuvent être substitués,

les radicaux alkoxy ayant de 1 à 12 atomes de carbone et les radicaux phénoxy,

les radicaux $—C_mH_{2m}—OH$ et $—C_mH_{2m}OR$ où m est un nombre entier compris entre 1 et 12 ($1 \leq m \leq 12$) et où R est un radical alkyle ayant de 1 à 12 atomes de carbone ou un radical phényle,

les radicaux alkylthio ayant de 1 à 12 atomes de carbone et les radicaux phénylthio,

les radicaux $C_pH_{2p+1-q}F_q$, p étant compris entre 1 et 4 ($1 \leq p \leq 4$) et q étant compris entre 3 et 9 ($3 \leq q \leq 9$) comme $—CF_3$ et $—CH_2—CF_3$ par exemple

les radicaux 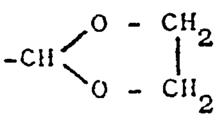 et 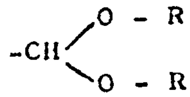 où

R est un radical alkyle ayant de 1 à 12 atomes de carbone ou un radical phényle,

les radicaux Cl, F, et Br,

et les radicaux $—NO_2$, $—SO_3M$, $—CN$, $—CO_2M$, $—CO_2R$, $—COR$, $—COH$ où M représente un métal alcalin et où R représente un radical alkyle ayant de 1 à 12 atomes de carbone ou un radical phényle.

n est un nombre entier pouvant être égal à 1, 2 ou 3 ($1 \leq n \leq 3$).

Les réactifs anioniques oxygénés ou soufrés que l'on peut mettre en œuvre selon le procédé de l'invention ont pour formule générale :

$$R_7 — A^-M^+ \qquad (III)$$

dans laquelle

$R_7$ représente un radical choisi parmi le groupe comprenant :

les radicaux alkyle linéaires ou ramifiés et les radicaux cycloalkyle ayant de 1 à 12 atomes de carbone et éventuellement substitués,

les radicaux aryle éventuellement substitués.

A représente l'oxygène ou le soufre,

$M^+$ représente un cation monovalent ou bivalent dérivé d'un métal alcalin ou alcalino-terreux ou le cation ammonium $NH_4^+$.

Les composés de formule III plus particulièrement, mais non exclusivement, visés par le procédé selon l'invention sont ceux pour lesquels $R_7$ représente un radical choisi parmi le groupe comprenant :

les radicaux alkyle linéaires ou ramifiés et les radicaux cycloalkyle ayant de 1 à 6 atomes de carbone et éventuellement substitués,

les radicaux phényle et naphtyle éventuellement substitués par au moins un des radicaux suivants : les radicaux alkyle ayant de 1 à 6 atomes de carbone, les radicaux phényle, les radicaux halogéno, nitro, cyano, amido, amino, les radicaux alkoxy ayant de 1 à 6 atomes de carbone, phényloxy, alkylamino ayant de 1 à 6 atomes de carbone, phénylamino, alkylamido ayant de 1 à 6 atomes de carbone, phénylamido.

On peut citer comme exemples de composés de formule II les composés suivants :

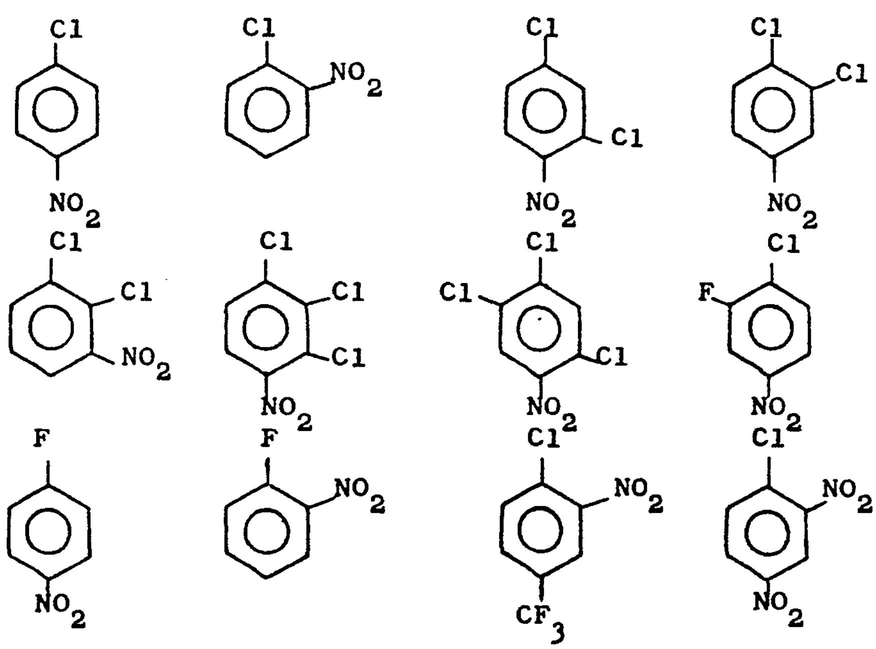

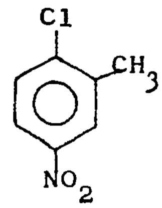
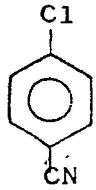
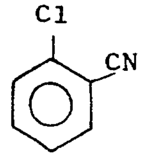
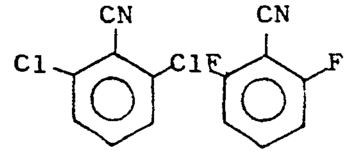

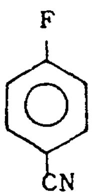
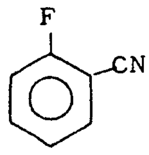
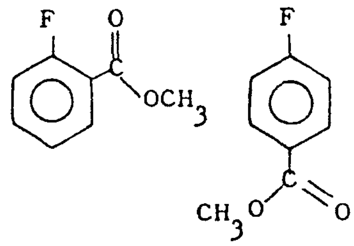

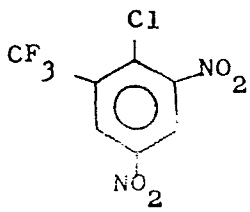
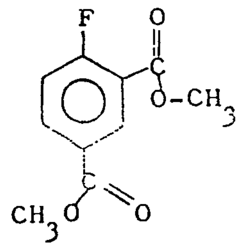
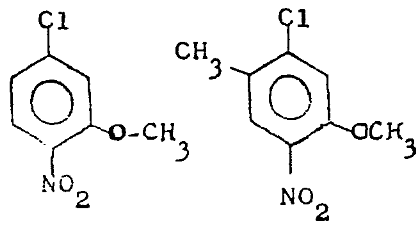

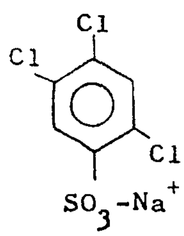
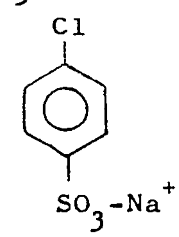
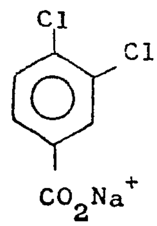
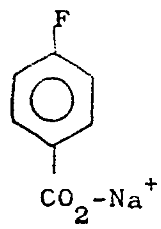

On peut citer comme exemples de composés de formule III les sels alcalins ou d'ammonium des composés suivants : les alcools comme le méthanol, l'éthanol, l'isopropanol, le butanol, les cycloalcools comme le cyclohexanol, le furfurol, les phénols comme le phénol, les alkylphénols comme l'o, p et m-crésol, l'isopropyl-2 méthyl-4 phénol et l'isopropyl-2 méthyl-5 phénol, le dodécylphénol, le nonylphénol, les aryls phénols comme le paraphénylphénol, les monohalogénophénols comme l'o, p et m-chlorophénol et les composés bromés iodés et fluorés correspondants, les polyhalogénophénols comme les dichlorophénols, les trichlorophénols, les tétrachlorophénols et le pentachlorophénol, les dihalogénophénols « mixtes» comme le chloro-3 bromo-4 phénol, le chloro-3 fluoro-4 phénol, le chloro-3 fluoro-5 phénol et les composés équivalents, les halogénoalkylphénols comme le trifluorométhyl-3 phénol et le trifluorométhyl-4 phénol, les alkylhalogénophénols comme le méthyl-2 chloro-4 phénol, le diméthyl-2,4 chloro-5 phénol, les aminophénols comme l'amino-3 phénol, l'amino-4 phénol, le méthyl-2 amino-4 phénol et le NN diméthyl amino-2 phénol, les cyanophénols comme le cyano-2 phénol et le cyano-4 phénol, les nitrophénols comme les o-, p- et m-nitrophénols, le méthyl-2 nitro-3 phénol, le méthyl-2 nitro-4 phénol, le dinitro-2,4 phénol, les amidophénols comme les o-p et m-amidophénols, les alkoxyphénols comme le méthoxy-3 phénol, le méthoxy-2 phénol, le méthoxy-4 phénol, les phénoxyphénols comme l'o-m-p-phénoxyphénol, les alkylamidophénols comme le diméthylamido-2 phénol, les thioalcools comme le méthylmercaptan, l'éthylmercaptan, les thiophénols comme le p-chlorothiophénol, le p-aminothiophénol, le méthyl-2 thiophénol, le méthyl-3 thiophénol, le méthyl-4 thiophénol, le diméthyl-2,4 thiophénol, les mercaptobenzothiazoles.

Le choix de l'agent séquestrant le plus adapté à la mise en œuvre du procédé selon l'invention doit être fait en tenant compte de la taille du cation $M^+$ (composé de formule III). Plus la taille du cation sera importante, plus le nombre d'atomes d'oxygène contenus dans la molécule de l'agent séquestrant devra être élevé. C'est ainsi que si on utilise un phénolate de potassium, on préfèrera utiliser la tris(trioxa-3, 6, 9 décyl)amine alors qu'avec le sel de sodium correspondant : la tris(dioxa-3,6 heptyl)amine sera préférée.

Le tiers solvant, lorsqu'on en utilise un, doit répondre à un certain nombre de conditions : il faut d'abord qu'il solubilise l'agent séquestrant (ce dernier est soluble dans la plupart des solvants usuels) ; il faut aussi qu'il soit inerte chimiquement vis-à-vis des sels à dissoudre. Il faut aussi noter que pour obtenir la meilleure mise en œuvre du procédé selon l'invention, plus le solvant choisi aura un caractère apolaire marqué, plus l'agent séquestrant devra avoir un caractère lipophyle marqué (c'est-à-dire plus l'agent séquestrant devra contenir d'atomes de carbone).

On peut par exemple utiliser comme tiers solvant l'acétonitrile, la N-méthylpyrrolidone, le chlorobenzène, l'odichlorobenzène, le diméthylsulfoxyde, le diphényléther, le dioxanne, les polyéthers d'éthylène-glycol (communément appelés « glymes »).

On peut utiliser les composés II et III en quantités stœchiométriques ou en excès par rapport à la quantité stœchiométrique. Selon un mode de réalisation préféré, on utilise un excès de 20 % par rapport à la stœchiométrie de l'un ou l'autre des composés II et III.

La quantité d'amine de formule I utilisée peut être comprise entre 1 et 100 moles pour 100 du composé de formule III. On préfère utiliser entre 1 et 15 moles d'amine pour 100 moles de composé III.

Lorsqu'on utilise un tiers solvant, la quantité de ce dernier mise en œuvre est telle qu'il contienne de 10 à 500 % de son poids de composé de formule III.

Le procédé selon l'invention est mis en œuvre à une température comprise entre 50 °C et 200 °C, de préférence entre 80 °C et 160 °C.

La pression n'est pas critique. On opère généralement sous pression atmosphérique bien que des pressions inférieures ou supérieures ne soient pas exclues.

Les composés obtenus selon le procédé de l'invention ont pour formules générales, les formules générales IV suivantes :

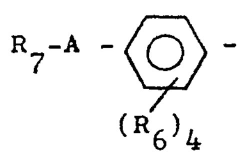 (IVa)

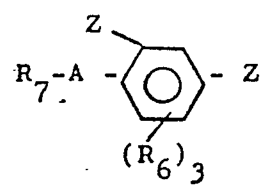 (IVc)

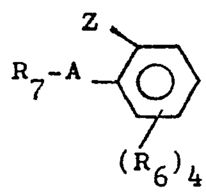 (IVb)

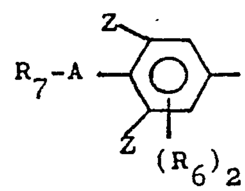 (IVd)

dans lesquelles $R_6$, $R_7$, A et Z ont les significations précédentes.

On peut citer comme exemples de composés répondant à l'une des formules IVa-d les composés suivants :

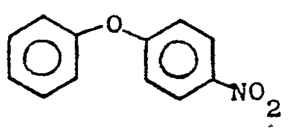

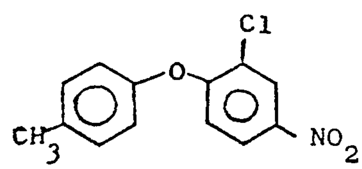

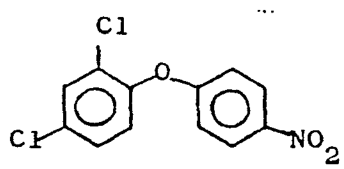

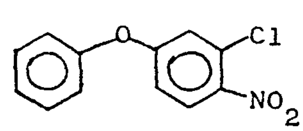

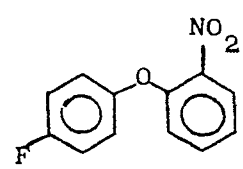

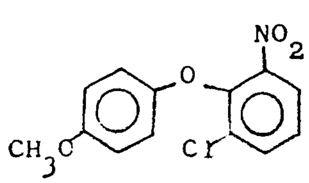

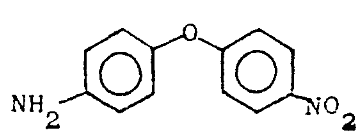

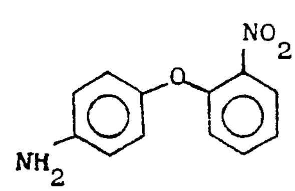

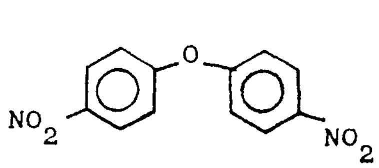

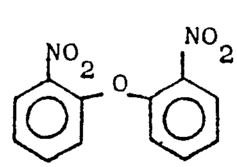

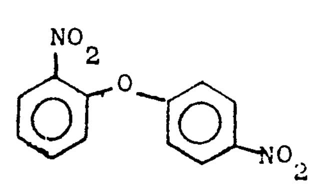

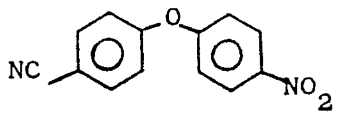 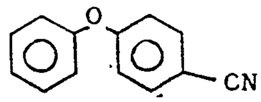 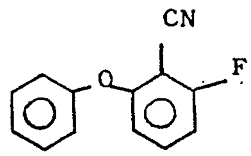

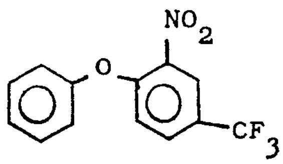 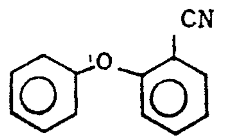 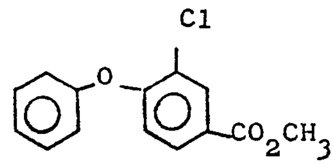

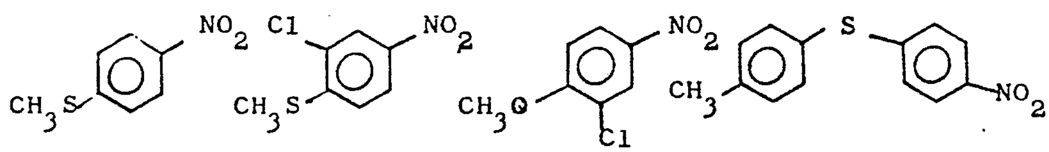

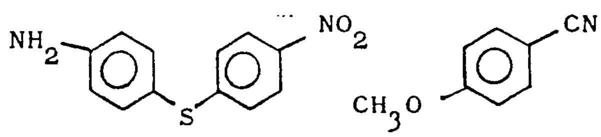 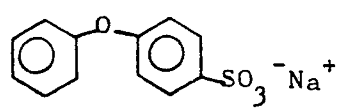

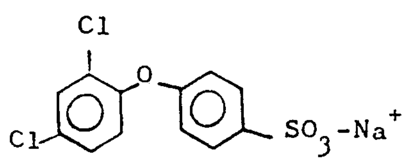 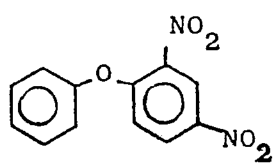 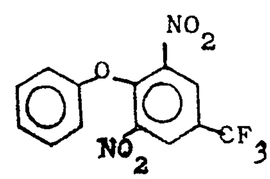

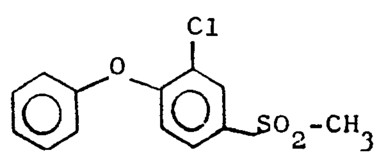

Ces composés sont utiles notamment en tant qu'intermédiaires pour la synthèse de composés organiques utilisables comme agents phytosanitaires.

Les agents séquestrants de formule I utilisés dans le procédé selon l'invention peuvent être préparés par condensation d'un sel de formule :

$$R_5 ( O - \underset{}{\overset{R_4}{|}}{CH} - \overset{R_3}{|}{CH} )_n -O-M$$

où $R_3$, $R_4$, $R_5$ et n ont la signification précédente et où M représente un atome de métal alcalin choisi parmi le sodium, le potassium et le lithium, soit sur une amine de formule générale :

$$N-(\overset{R_1}{\overset{|}{C}H} - \overset{R_2}{\overset{|}{C}H} - X)_3$$

dans laquelle $R_1$ et $R_2$ ont la signification précédente et X représente le chlore ou le brome, soit sur le chlorhydrate ou le bromhydrate correspondant.

Le rapport molaire sel de métal alcalin/amine est compris entre environ 3 et environ 5.

L'opération de condensation est réalisée à une température comprise entre 100 et 150 °C pendant 1 à 15 h en présence d'un solvant qui peut être par exemple le chlorobenzène ou de préférence le monoalkyléther d'éthylène glycol de formule $R_5—(O—CHR_4—CHR_3)_n—OH$.

On opère de préférence de telle sorte qu'on ait une solution contenant de 2 à 5 moles de sel de métal alcalin par litre de solvant.

Le mélange en fin de réaction contient principalement l'amine tertiaire de formule :

$$N-\left[\overset{R_1}{\overset{|}{C}H}-\overset{R_2}{\overset{|}{C}H}-O-(\overset{R_3}{\overset{|}{C}H}-\overset{R_4}{\overset{|}{C}H}-O)_n-R_5\right]_3$$

mais contient aussi en faible proportion de l'amine secondaire correspondant :

$$HN-\left[\underset{R_1}{\underset{|}{C}H}-\underset{R_2}{\underset{|}{C}H}-O-(\underset{R_3}{\underset{|}{C}H}-\underset{R_4}{\underset{|}{C}H}-O)_n-R_5\right]_2$$

et des traces d'amine primaire :

$$H_2N-\left[\underset{R_1}{\underset{|}{C}H}-\underset{R_2}{\underset{|}{C}H}-O-(\underset{R_3}{\underset{|}{C}H}-\underset{R_4}{\underset{|}{C}H}-O)_n-R_5\right]$$

Les amines tertiaires, secondaires et primaires sont généralement respectivement dans le rapport 90 : 8 : 2 après distillation.

On peut utiliser dans le procédé selon l'invention directement le mélange ci-dessus obtenu après première distillation, c'est-à-dire contenant les trois types d'amines.

On préfère pour une meilleure mise en œuvre de l'invention effectuer une distillation plus poussée du mélange ci-dessus afin d'obtenir une amine tertiaire sensiblement pure.

D'autres caractéristiques et avantages de l'invention apparaîtront plus clairement à la lecture des exemples qui vont suivre. Ces exemples ne sauraient en aucune manière être considérés comme une limitation de l'invention.

## Exemple 1

Réaction de $\varphi O^-Na^+$ avec le paranitrochlorobenzène pour obtenir le paraphénoxynitro benzène de formule :

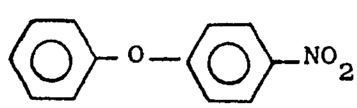

en présence de tris(dioxa-3, 6 heptyl)amine dans le dichlorobenzène.

Dans un ballon tricol de 500 ml équipé d'un agitateur mécanique, d'un thermomètre et d'un réfrigérant ascendant, on introduit successivement 100 $cm^3$ de chlorobenzène, 32 g de paranitrochlorobenzène (0,2 mole) 23 g de phénate de sodium (0,2 mole) et 3,7 g de tris(dioxa-3, 6 octyl)amine (0,01 mole). Le mélange est agité et chauffé à 130 °C pèndant 9 h. Le rendement de la réaction est de 95 % en para-phénoxy-nitrobenzène isolé.

Exemple comparatif

Dans les mêmes conditions opératoires, en l'absence de tris(dioxa-3, 6 octyl)amine le rendement de la réaction est de 3 %.

Exemple 2

Réaction du dichloro 2, 4 phénate de sodium, de formule :

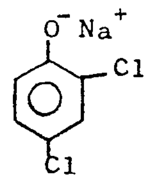

avec le paranitrochlorobenzène pour obtenir le para(dichloro-2, 4 phénoxy)nitrobenzène, de formule :

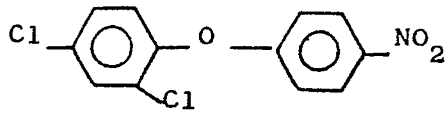

en présence de tris(dioxa-3, 6 heptyl)amine dans le dichlorobenzène.

Dans un ballon tricol de 500 ml équipé d'un agitateur mécanique, d'un thermomètre et d'un réfrigérant ascendant, on introduit successivement 100 $cm^3$ de monochlorobenzène, 15,7 g de parachloronitrobenzène (0,1 mole), 27,8 g de dichloro-2,4 phénate de sodium (0,16 mole) et 2,3 g de tris(dioxa-3, 6 heptyl)amine (0,007 mole). Le mélange est agité et chauffé à reflux du chlorobenzène pendant 12 h. Le rendement de la réaction est de 68 %.

Exemple comparatif

En l'absence de la tris(dioxa-3, 6 heptyl)amine le rendement est de 8 %.

Exemple 3

Réaction du paranitrophénate de potassium de formule :

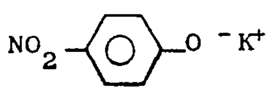

avec le parafluoronitrobenzène pour obtenir le dinitro-4, 4' diphényléther de formule :

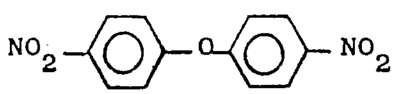

en présence de tris(trioxa-3, 6, 9 décyl)amine dans l'o-dichlorobenzène.

Dans un ballon tricol de 500 ml équipé d'un agitateur mécanique, d'un thermomètre et d'un réfrigérant ascendant on introduit successivement 200 $cm^3$ d'orthodichlorobenzène, 14,1 g de parafluoro-nitrobenzène (0,1 mole), 17,7 g de paranitrophénate de potassium (0,1 mole) et 4,55 g de tris(trioxa-3, 6, 9 décyl)amine (0,01 mole). Le mélange est agité et chauffé à reflux de l'ortho-dichlorobenzène pendant 10 h. Le rendement de la réaction est de 87 %.

Exemple 4

Réaction du paranitrophénate de potassium avec l'ortho-nitrofluorobenzène de formule :

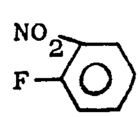

pour obtenir le dinitro-2, 4' diphényléther de formule :

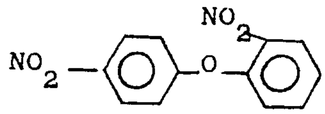

en présence de tris(trioxa-3, 6, 9 décyl)amine dans l'orthodichlorobenzène.

Dans un ballon tricol de 500 ml équipé d'un agitateur mécanique, d'un thermomètre et d'un réfrigérant ascendant, on introduit successivement 200 $cm^3$ d'ortho-dichlorobenzène, 14,1 g d'orthofluoronitrobenzène (0,1 mole) 17,7 g de paranitrophénate de potassium (0,1 mole) et 4,55 g de tris(trioxa-3, 6, 9 décyl)amine (0,01 mole). Le mélange est agité et chauffé à reflux de l'orthodichlorobenzène pendant 10 h. Après refroidissement, on élimine les sels puis on évapore le solvant. Le rendement de la réaction est de 85 %.

Exemple 5

Réaction de l'orthonitrophénate de potassium de formule :

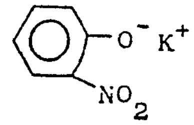

et de l'orthonitrofluorobenzène pour obtenir le dinitro-2, 2' diphényléther de formule :

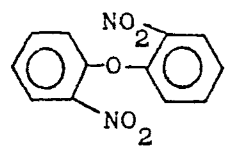

en présence de tris(trioxa-3, 6, 9 décyl)amine dans l'o-dichlorobenzène.

Dans un ballon tricol de 500 ml équipé d'un agitateur mécanique, d'un thermomètre et d'un réfrigérant ascendant, on introduit successivement 200 $cm^3$ d'orthodichlorobenzène, 14,1 g d'ortho-fluoro-nitrobenzène (0,1 mole) 17,7 g d'orthonitrophénate de potassium (0,1 mole) et 4,55 g de tris(trioxa-3, 6, 9 décyl)amine, (0,01 mole). Le mélange est agité et chauffé à reflux de l'orthodichlorobenzène pendant 8 h. Après refroidissement, on élimine les sels puis on évapore le solvant. Le rendement de la réaction est de 82 %.

Exemple 6

Réaction du paraaminophénate de sodium avec le paranitrochlorobenzène pour obtenir le para(amino-4 phénoxy)nitrobenzène de formule :

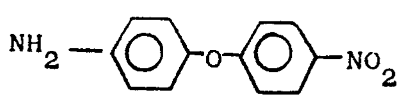

en présence de tris(dioxa-3, 6 heptyl)amine dans le chlorobenzène.

Dans un ballon tricol de 500 ml équipé d'un agitateur, mécanique, d'un thermomètre et d'un réfrigérant ascendant, on introduit successivement 100 $cm^3$ de chlorobenzène, 15,7 g de parachloronitrobenzène (0,1 mole), 13,1 g de paraaminophénate de sodium (0,1 mole) et 1,6 g de tris(dioxa-3, 6 heptyl)-amine (0,005 mole). Le mélange est agité et chauffé à 130 °C pendant 13 h, puis on filtre la solution obtenue à chaud. Après filtration, on ajoute 300 $cm^3$ d'hexane qui provoque la précipitation du para(amino-4 phénoxy)nitrobenzène. Le rendement de la réaction est de 83 %.

Exemple 7

Réaction du thiométhylate de sodium de formule : $CH_3S^-Na^+$ avec le paranitrochlorobenzène pour obtenir le parathiométhoxy nitrobenzène de formule :

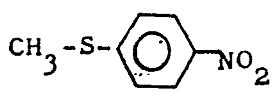

en présence de tris(dioxa-3, 6 heptyl)amine dans le chlorobenzène.

Dans un ballon tricol de 2 litres équipé d'un agitateur mécanique, d'un thermomètre et d'un condenseur ascendant, on introduit successivement 1 litre de chlorobenzène, 157 g de para-nitrochlorobenzène (1 mole) 140 g de thiométhylate de sodium (2 moles) et 32 g de tris(dioxa-3, 6 heptyl)amine (0,1 mole). Le mélange est agité et chauffé pendant 2 h à reflux du chlorobenzène puis refroidi. Les sels formés ainsi que le thiométhylate de sodium non transformé sont éliminés par filtration et le chlorobenzène est évaporé. Le thiométhoxy-nitrobenzène est distillé. Le rendement de la réaction est de 72 %.

### Exemple 8

Réaction du phénate de potassium avec le parachloro benzonitrile pour obtenir le paraphénoxybenzonitrile de formule :

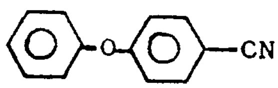

en présence de tris(trioxa-3, 6, 9 décyl)amine dans l'o-dichlorobenzène.

Dans un ballon tricol de 500 ml équipé d'un agitateur mécanique, d'un thermomètre et d'un réfrigérant ascendant, on introduit successivement 200 $cm^3$ d'orthodichlorobenzène, 27,5 g de parachlorobenzonitrile (0,2 mole), 29,04 g de phénate de potassium (0,22 mole) et 4,55 g de tris(trioxa-3, 6, 9 décyl)-amine (0,01 mole). On agite et on chauffe le mélange réactionnel à reflux de l'orthodichlorobenzène pendant 8 h. On élimine les sels formés par filtration et le solvant par distillation. Le rendement de la réaction est de 85 %.

### Exemple comparatif

Dans les mêmes conditions opératoires, en l'absence de la tris(trioxa-3, 6, 9 décyl)amine, le rendement est seulement de 3 %.

### Exemple 9

Réaction du phénate de sodium avec le nitro-3 chloro-4 trifluorométhylbenzène pour obtenir le nitro-3 phénoxy-4 trifluorométhylbenzène de formule :

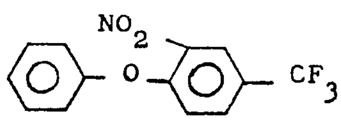

en présence de tris(dioxa-3, 6 heptyl)amine dans le chlorobenzène.

Dans un ballon tricol de 500 ml équipé d'un agitateur mécanique, d'un thermomètre et d'un réfrigérant ascendant, on introduit successivement 200 $cm^3$ de chlorobenzène, 22,5 g de nitro-3 chloro-4 trifluorométhylbenzène (0,1 mole), 13 g de phénate de sodium (0,11 mole) et 3,2 g de tris(dioxa-3, 6 heptyl)-amine (0,01 mole).

Le mélange est agité et chauffé à reflux du chlorobenzène pendant 4 h. Après refroidissement, on filtre les sels puis on évapore le solvant. Le rendement de la réaction est de 92 %.

### Exemple comparatif

En l'absence de tris(dioxa-3, 6 heptyl)amine, le rendement de la réaction est de 18 %.

### Exemple 10

Préparation de la tris(dioxa-3, 6 heptyl)amine

a) Dans un ballon tricol d'un litre, muni d'un agitateur mécanique, d'un thermomètre, d'un réfrigérant, on introduit 380 g de méthoxy-2 éthanol (5 moles). On ajoute 23 g de sodium (1 mole) en 3 h en maintenant la température de mélange à 40 °C.

b) Au mélange précédent, on ajoute 51,6 g de chlorhydrate de tris(chloro-2 éthyl)amine (soit 0,215 mole). On chauffe alors le mélange à reflux du méthoxy-2 éthanol (125 °C) pendant 12 h, puis on distille le solvant sous pression réduite. Le méthoxy-2 éthanolate de sodium en excès est neutralisé par addition de 11,6 $cm^3$ d'HCl aqueux (10 N). Le chlorure de sodium est filtré et la solution est distillée.

Exemple 11

Préparation de la tris(trioxa-3, 6, 9 décyl)amine

Dans un ballon tricol d'un litre équipé d'un agitateur mécanique, d'un condenseur et d'un thermomètre, on introduit 600 g d'éther monométhylique du diéthylène glycol (dioxa-3, 6 heptanol-1) soit 5 moles puis 23 g de sodium (1 mole) par petites fractions afin de former le dioxa-3, 6 heptanolate de sodium.

Lorsque le sodium est totalement transformé, on ajoute alors 51,8 g de chlorhydrate de la tris(chloro-2 éthyl)amine (soit 0,215 mole). Le mélange est chauffé à 130 °C pendant 8 h sous agitation puis refroidi et l'excès d'alcoolate de sodium neutralisé par une solution aqueuse d'acide chlorhydrique à 10 %. Le dioxa-3, 6 heptanol-1 est éliminé par distillation à 130 °C sous 20 mmHg. Le mélange obtenu est filtré afin d'éliminer le chlorure de sodium puis le produit est distillé. On obtient ainsi 83 g de tris(trioxa-3, 6, 9 décyl)amine qui distille à 189 °C sous 0,1 mmHg.

Les autres agents séquestrants utilisés dans le cadre de la présente invention peuvent être préparés d'une façon similaire.

**Revendications**

1. Procédé de préparation de composés benzéniques par réaction d'un halogénobenzène comportant un groupement électroattracteur en ortho et/ou para de l'halogène avec un composé de formule $RO^-M^+$ ou $RS^-M^+$ dans laquelle R est un reste hydrocarboné et $M^+$ un cation monovalent ou bivalent dérivé d'un métal alcalin ou alcalino-terreux ou le cation ammonium caractérisé en ce que la réaction a lieu en présence d'au moins un agent séquestrant de formule :

$$N{-}[CHR_1{-}CHR_2{-}O{-}(CHR_3{-}CHR_4{-}O)_n{-}R_5]_3 \quad (I)$$

dans laquelle n est un nombre entier supérieur ou égal à 0 et inférieur ou égal à 10 ($0 \leq n \leq 10$), $R_1$, $R_2$, $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $-C_mH_{2m}-C_6H_5$ ou $C_mH_{2m+1}-C_6H_4-$, où m est compris entre 1 et 12.

2. Procédé selon la revendication 1, caractérisé en ce que dans la formule (I), $R_1$, $R_2$, $R_3$ et $R_4$ représentent un atome d'hydrogène ou un radical méthyle.

3. Procédé selon la revendication 1, caractérisé en ce que dans la formule (I), n est un nombre entier supérieur ou égal à 0 et inférieur ou égal à 6.

4. Procédé selon la revendication 1, caractérisé en ce que dans la formule (I), $R_5$ représente un radical alkyle ayant de 1 à 4 atomes de carbone.

5. Procédé selon la revendication 1, caractérisé en ce que dans la formule (I), $R_1$, $R_2$, $R_3$ et $R_4$ identiques ou différents représentent un atome d'hydrogène ou un radical méthyle, n est un nombre entier supérieur ou égal à 0 et inférieur ou égal à 6 et $R_5$ représente un radical alkyle ayant de 1 à 4 atomes de carbone.

6. Procédé selon la revendication 5, caractérisé en ce que l'agent séquestrant de formule (I) est la tris(dioxa-3, 6 heptyl)amine de formule : $N{-}(CH_2{-}CH_2{-}O{-}CH_2{-}CH_2{-}O{-}CH_3)_3$.

7. Procédé selon la revendication 5, caractérisé en ce que l'agent séquestrant de formule (I) est la tris(trioxa-3, 6, 9 décyl)amine de formule : $N{-}(CH_2{-}CH_2{-}O{-}CH_2{-}CH_2{-}O{-}CH_2{-}CH_2{-}O{-}CH_3)_3$.

8. Procédé selon la revendication 5, caractérisé en ce que l'agent séquestrant de formule (I) est la tris(dioxa-3, 6 octyl)amine de formule : $N{-}(CH_2{-}CH_2{-}O{-}CH_2{-}CH_2{-}O{-}C_2H_5)_3$.

9. Procédé selon l'une quelconque des revendications précédentes, caractérisé en ce qu'on opère en présence d'un solvant choisi parmi le chlorobenzène, l'o-dichlorobenzène, le diphényléther, le dioxanne, les polyéthers d'éthylèneglycol, la N-méthylpyrrolidone, le diméthylsulfoxyde.

10. Procédé selon l'une quelconque des revendications 1 à 9, caractérisé en ce qu'on utilise l'agent séquestrant de formule (I) en quantité telle que le rapport molaire de l'agent séquestrant au composé $RO^-M^+$ ou $RS^-M^+$ est compris entre 1/100 et 1.

11. Procédé selon la revendication 10, caractérisé en ce que le rapport molaire de l'agent séquestrant au composé $RO^-M^+$ ou $RS^-M^+$ est compris entre 1/100 et 15/100.

12. Procédé selon la revendication 9, caractérisé en ce que le solvant est en quantité telle qu'il contienne de 10 à 500 % de son poids de composé $RO^-M^+$ ou $RS^-M^+$.

13. Procédé selon l'une quelconque des revendications précédentes, caractérisé en ce qu'on opère en utilisant un excès d'environ 20 % par rapport à la stœchiométrie de l'halogénobenzène ou du composé $RO^-M^+$ ou $RS^-M^+$.

14. Procédé selon l'une quelconque des revendications précédentes,caractérisé en ce qu'on opère à une température comprise entre 50 °C et 200 °C.

15. Procédé selon la revendication 14, caractérisé en ce que la température est comprise entre 80 °C et 160 °C.

**Claims**

1. Process for the preparation of benzene compounds by the reaction of a halogenobenzene containing an electron-attracting group in the ortho-position and/or paraposition to the halogen with a compound of the formula $RO^-M^+$ or $RS^-M^+$, in which R is a hydrocarbon radical and $M^+$ is a monovalent or divalent cation derived from an alkali metal or alkaline earth metal, or the ammonium cation, characterised in that the reaction takes place in the presence of at least one sequestering agent of the formula :

$$N-[CHR_1-CHR_2-O-(CHR_3-CHR_4-O)_n-R_5]_3 \qquad (I)$$

in which n is an integer which is greater than or equal to 0 and less than or equal to 10 ($0 \leq n \leq 10$), $R_1$, $R_2$, $R_3$ and $R_4$, which are identical or different, represent a hydrogen atom or an alkyl radical having from 1 to 4 carbon atoms, and $R_5$ represents an alkyl or cycloalkyl radical having from 1 to 12 carbon atoms, a phenyl radical or a radical $-C_mH_{2m}-C_6H_5$ or $C_mH_{2m+1}-C_6H_4-$, in which m is between 1 and 12.

2. Process according to Claim 1, characterised in that, in the formula (I), $R_1$, $R_2$, $R_3$ and $R_4$ represent a hydrogen atom or a methyl radical.

3. Process according to Claim 1, characterised in that, in the formula (I), n is an integer which is greater than or equal to 0 and less than or equal to 6.

4. Process according to Claim 1, characterised in that, in the formula (I), $R_5$ represents an alkyl radical having from 1 to 4 carbon atoms.

5. Process according to Claim 1, characterised in that, in the formula (I), $R_1$, $R_2$, $R_3$ and $R_4$, which are identical or different, represent a hydrogen atom or a methyl radical, n is an integer which is greater than or equal to 0 and less than or equal to 6, and $R_5$ represents an alkyl radical having from 1 to 4 carbon atoms.

6. Process according to Claim 5, characterised in that the sequestering agent of the formula (I) is tris-(3, 6-dioxaheptyl)-amine of the formula : $N-(CH_2-CH_2-O-CH_2-CH_2-O-CH_3)_3$.

7. Process according to Claim 5, characterised in that the sequestering agent of the formula (I) is tris-(3, 6, 9-trioxadecyl)-amine of the formula : $N-(CH_2-CH_2-O-CH_2-CH_2-O-CH_2-CH_2-O-CH_3)_3$.

8. Process according to Claim 5, characterised in that the sequestering agent of the formula (I) is tris-(3, 6-dioxaoctyl)-amine of the formula : $N-(CH_2-CH_2-O-CH_2-CH_2-O-C_2H_5)_3$.

9. Process according to any one of the preceding claims, characterised in that the reaction is carried out in the presence of a solvent chosen from amongst chlorobenzene, o-dichlorobenzene, diphenyl ether, dioxane, polyethers of ethylene glycol, N-methylpyrrolidone and dimethyl sulphoxide.

10. Process according to any one of Claims 1 to 9, characterised in that the sequestering agent of the formula (I) is used in an amount such that the molar ratio of the sequestering agent to the compound $RO^-M^+$ or $RS^-M^+$ is between 1/100 and 1.

11. Process according to Claim 10, characterised in that the molar ratio of the sequestering agent to the compound $RO^-M^+$ or $RS^-M^+$ is between 1/100 and 15/100.

12. Process according to Claim 9, characterised in that the solvent is in an amount such that it contains from 10 to 500 % of its weight of the compound $RO^-M^+$ or $RS^-M^+$.

13. Process according to any one of the preceding claims, characterised in that the reaction is carried out using an approximately 20 % excess, relative to stoichiometry, of the halogenobenzene or of the compound $RO^-M^+$ or $RS^-M^+$.

14. Process according to any one of the preceding claims, characterised in that the reaction is carried out at a temperature of between 50 °C and 200 °C.

15. Process according to Claim 14, characterised in that the temperature is between 80 °C and 160 °C.

**Ansprüche**

1. Verfahren zur Herstellung von Benzolverbindungen durch Umsetzung eines Halogenbenzols mit einer Elektronen anziehenden Gruppe in Ortho- und/oder Parastellung zum Halogen mit einer Verbindung der Formel $RO^-M^+$ oder $RS^-M^+$, in der R ein Kohlenwasserstoffrest und M ein einwertiges oder zweiwertiges Alkali- oder Erdalkalikation oder das Ammoniumion ist, dadurch gekennzeichnet, daß die Reaktion in Gegenwart wenigstens eines Sequestrierungsmittels der Formel :

$$N-[CHR_1-CHR_2-O-(CHR_3-CHR_4-O)_n-R_5]_3 \qquad (I)$$

durchgeführt wird, in der n ein ganze Zahl größer oder gleich 0 und kleiner oder gleich 10 ($0 \leq n \leq 10$) ist, $R_1$, $R_2$, $R_3$ und $R_4$ gleich oder verschieden ein Wasserstoffatom oder einen Alkylrest mit 1 bis 4 Kohlenstoffatomen bedeuten und $R_5$ einen Alkyl- oder Cycloalkylrest mit 1 bis 12 Kohlenstoffatomen, einen Phenylrest oder einen Rest $-C_mH_{2m}-C_6H_5$ oder $C_mH_{2m+1}-C_6H_4-$ bedeutet, wobei m zwischen 1 und 12 liegt.

2. Verfahren nach Anspruch 1, dadurch gekennzeichnet, daß in der Formel (I) $R_1$, $R_2$, $R_3$ und $R_4$ ein Wasserstoffatom oder einen Methylrest bedeuten.

3. Verfahren nach Anspruch 1, dadurch gekennzeichnet, daß in der Formel (I) n eine ganze Zahl gleich oder größer 0 und kleiner oder gleich 6 ist.

4. Verfahren nach Anspruch 1, dadurch gekennzeichnet, daß in der Formel (I) $R_5$ einen Alkylrest mit 1 bis 4 Kohlenstoffatomen bedeutet.

5. Verfahren nach Anspruch 1, dadurch gekennzeichnet, daß in der Formel (I) $R_1$, $R_2$, $R_3$ und $R_4$ gleich oder verschieden ein Wasserstoffatom oder einen Methylrest bedeuten, n eine ganze Zahl größer oder gleich 0 und kleiner oder gleich 6 ist und $R_5$ einen Alkylrest mit 1 bis 4 Kohlenstoffatomen bedeutet.

6. Verfahren nach Anspruch 5, dadurch gekennzeichnet, daß das Sequestrierungsmittel der Formel (I) Tris(3, 6-dioxaheytyl)amin der Formel : $N—(CH_2—CH_2—O—CH_2—CH_2—O—CH_3)_3$ ist.

7. Verfahren nach Anspruch 5, dadurch gekennzeichnet, daß das Sequestrierungsmittel der Formel (I) Tris(3, 6,-trioxadecyl)amin der Formel : $N—(CH_2—CH_2—O—CH_2—CH_2—O—CH_2—CH_2—O—CH_3)_3$ ist.

8. Verfahren nach Anspruch 5, dadurch gekennzeichnet, daß das Sequestrierungsmittel der Formel (I) das Tris(3, 6,-dioxa-octyl)amin der Formel : $N—(CH_2—CH_2—O—CH_2—CH_2—O—C_2H_5)_3$ ist.

9. Verfahren nach einem der vorangehenden Ansprüche, dadurch gekennzeichnet, daß man in Gegenwart eines Lösungsmittels arbeitet, das unter Chlorbenzol, Orthodichlorbenzol, Diphenyläther, Dioxan, den Polyäthern des Äthylenglykols, N-Methylpyrrolidon und Dimethylsulfoxid gewählt ist.

10. Verfahren nach einem der Ansprüche 1 bis 9, dadurch gekennzeichnet, daß man das Sequestrierungsmittel der Formel (I) in einer solchen Menge verwendet, daß das Molverhältnis des Sequestrierungsmittels zur Verbindung $RO^-M^+$ oder $RS^-M^+$ zwischen 1 : 100 und 1 liegt.

11. Verfahren nach Anspruch 10, dadurch gekennzeichnet, daß das Molverhältnis des Sequestrierungsmittels zur Verbindung $RO^-M^+$ oder $RS^-M^+$ zwischen 1 : 100 und 15 : 100 liegt.

12. Verfahren nach Anspruch 9, dadurch gekennzeichnet, daß das Lösungsmittel in solcher Menge vorliegt, das es 10 bis 500 % seines Gewichts der Verbindung $RO^-M^+$ oder $RS^-M^+$ enthält.

13. Verfahren nach einem der vorangehenden Ansprüche, dadurch gekennzeichnet, daß man mit einem Überschuß von etwa 20 %, bezogen auf die stöchiometrische Menge des Halogenbenzols oder der Verbindung $RO^-M^+$ oder $RS^-M^+$ arbeitet.

14. Verfahren nach einem der vorangehenden Ansprüche, dadurch gekennzeichnet, daß man bei einer Temperatur zwischen 50 und 200 °C arbeitet.

15. Verfahren nach Anspruch 14, dadurch gekennzeichnet, daß die Temperatur zwischen 80 und 160 °C liegt.